# EUROPEAN PATENT APPLICATION

(11) **EP 1 754 711 A1**
(43) Date of publication of application: **21.02.2007**
(21) Application number: 05743753.5
(22) Date of filing: 26.05.2005
(51) Int. Cl.: C07H 19/23

(54) **PROCESS FOR PRODUCING INDOLOPYRROLOCARBAZOLE DERIVATIVE**

(30) Priority: 28.05.2004 JP 2004160193
(71) Applicant: BANYU PHARMACEUTICAL CO., LTD., Tokyo 102-8667 (JP)
(72) Inventor: IIDA, Takehiko, Hanecho, Okazaki-shi, Aichi 4440813 (JP); HIRAGA, Shouichi, Okazaki-shi, Aichi 4440874 (JP); TAKEZAWA, Akihiro, Okazaki-shi, Aichi 4440858 (JP); MASE, Toshiaki, Okazaki-shi, Aichi 4440822 (JP)
(74) Representative: Rollins, Anthony John
(86) International application number: PCT/JP2005/009674
(87) International publication number: WO 2005/116044

(57) **Abstract**

The present invention relates to an industrially preferable process for producing an indolopyrrolocarbazole derivative represented by the formula (I): 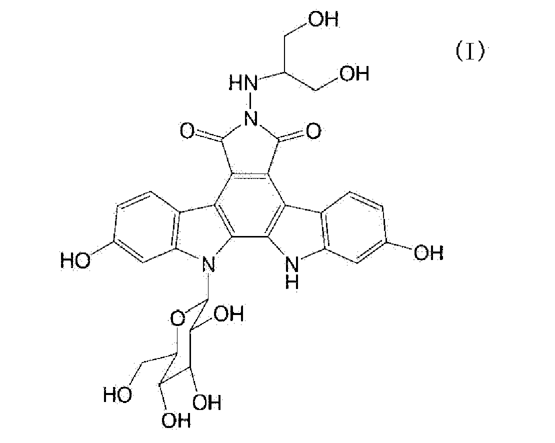 or a pharmaceutically acceptable salt thereof, which is useful as an anti-cancer agent.

The above process comprises treating a compound represented by the formula (V): 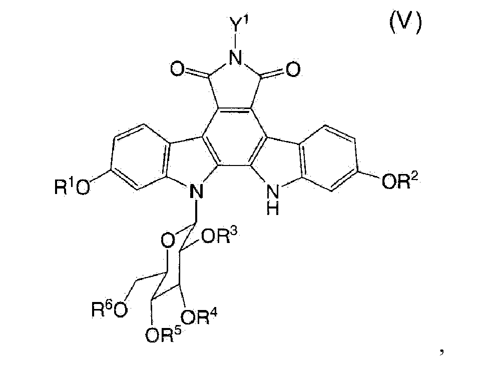 or a pharmaceutically acceptable salt thereof, a solvate thereof or a salt thereof, with a base in an inert solvent, followed by treatment with an acid, and further treating the resulting reaction solution with a base in an inert solvent and subsequently with an acid, and then reacting the resulting compound with an acid addition salt of hydrazine diol in the presence of an acid scavenger, followed by removal of protecting group(s) from the resulting compound.

## Description

### TECHNICAL FIELD

The present invention is useful in the pharmaceutical field. More specifically, the present invention relates to an industrially suitable process for producing an indolopyrrolocarbazole derivative (I) or a pharmaceutically acceptable salt thereof, which is useful in the pharmaceutical field, and also relates to a novel intermediate necessary for producing the same and a process for producing the same.

### BACKGROUND OF THE INVENTION

Mitsuru Ohkubo et al., Bioorganic & Medicinal Chemistry Letters, vol.9, P.3307-3312 (1999) discloses that an indolopyrrolocarbazole derivative represented by the formula (I): 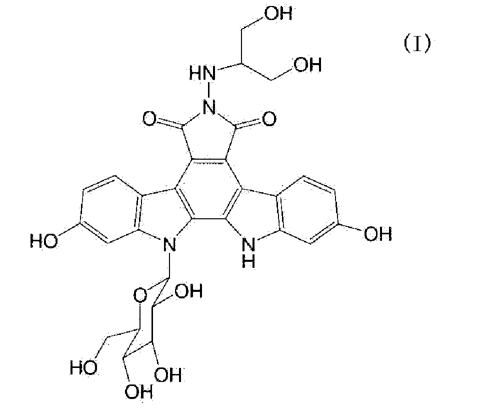 which is produced by the process of the present invention is a compound which has anti-cancer activity and is now clinically being tested.

In addition, regarding a process for producing the indolopyrrolocarbazole derivative (I), Japanese Patent No. 3038921 discloses a process shown by the following reaction scheme.

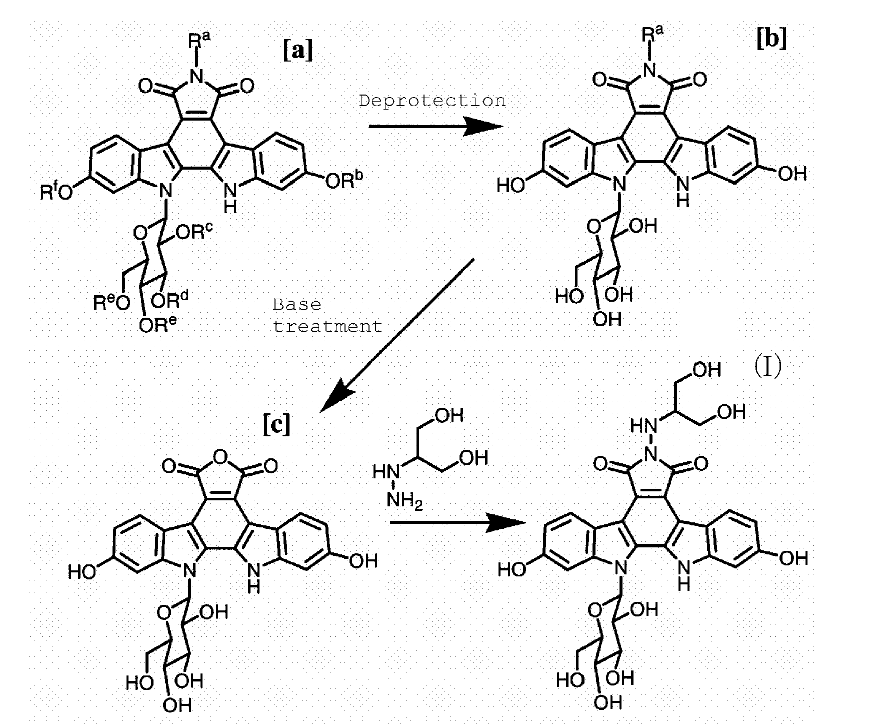

However, a compound [b] and a compound [c] have an unfavorable influence (e.g. skin reddening), even with a small amount, on a human body upon contact.

The above process includes a step of handling a physiologically highly active compound which is unfavorable to a human body as those compounds mentioned above, that is, the step of producing compound [b] and compound [c]. Therefore, in view of the following (A) to (C), such steps are not preferable for industrially producing a compound (I), and thus deletion of such steps is desired.
(A) Production facilities for the steps must be placed in a room specially equipped with a ventilation system for preventing leakage of the physiologically highly active compound.
(B) Construction fees and running costs for the room are high, and also costs for treatment of waste produced in the steps is high because of the presence of the highly active compound included therein.
(C) Since a worker engaged in the steps has to wear a protective clothing which covers the entire body while being fed with fresh air to breathe, physical burden on the worker is heavy, lowering work efficiency to about one half of the normal level.

In addition, regarding a glycosylation step in the process for producing an indolopyrrolocarbazole derivative (I), WO 02/36601 discloses a production process shown by the following reaction scheme.

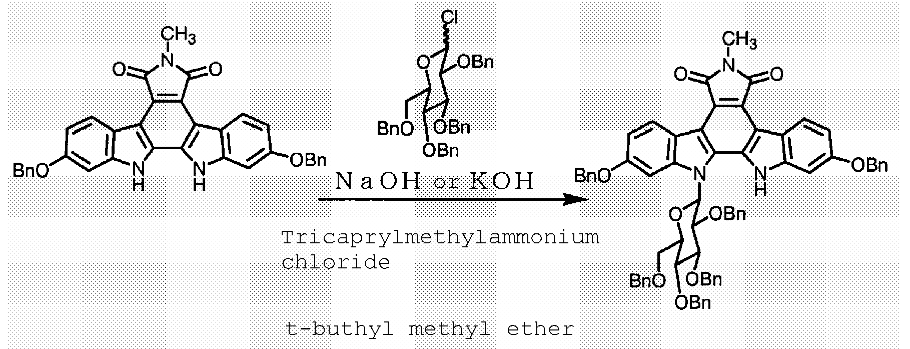

In the above reaction scheme, Bn represents a benzyl group.

As a production process solving an unfavorable point of the above industrial production of the compound (I) relating to the production process disclosed in Japanese Patent No. 3038921 (i.e. deletion of two steps handling a physiologically highly active compound), Japanese Patent No. 3388489 discloses a process for producing an indolopyrrolocarbazole derivative shown by the following reaction scheme: 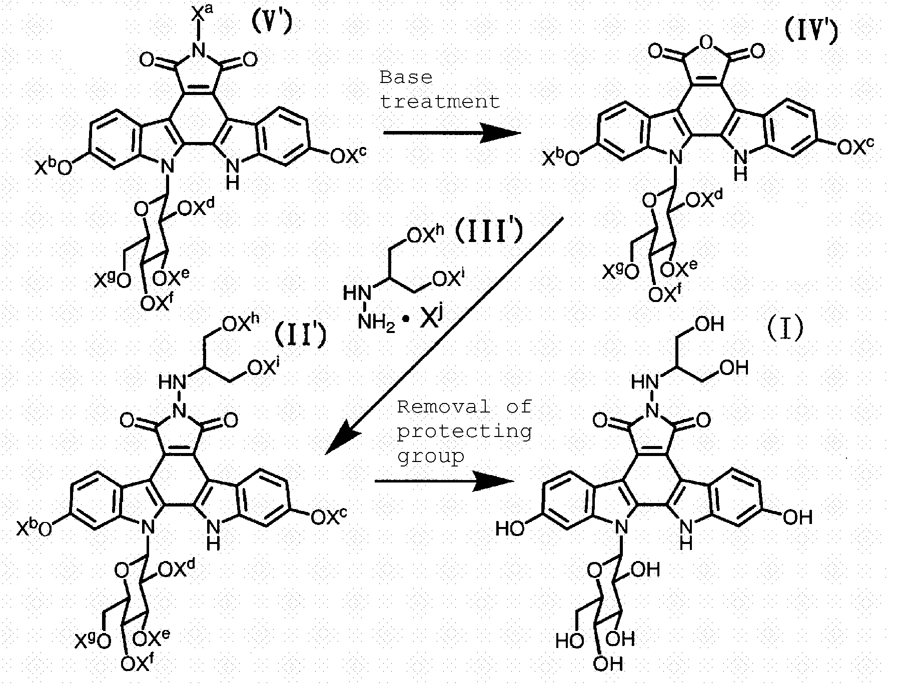 wherein X^{a} represents a hydrogen atom, a C₁-C₄ alkyl group, a phenyl group, a benzyloxymethyl group or an aralkyl group; X^{b}, X^{c}, X^{d}, X^{e}, X^{f} and X^{g} each independently represent a hydroxy protecting group; X^{h} and Xⁱ each independently represent a hydrogen atom or a hydroxy protecting group; and Xj represents an acid molecule.

### DISCLOSURE OF THE INVENTION

An object of the present invention is to improve a process for producing an indolopyrrolocarbazole derivative (I), which is disclosed in WO 02/36601 and Japanese Patent No. 3388489, into a more excellent production process as an industrial production process.

In other words, objects of the present invention are the following (i) and (ii), that is:
(i) improvement in yield of a compound (IV') and a compound (V'), and
(ii) reduction in the contents of impurities, which are produced as a byproduct in the step of producing a compound (IV') from a compound (V'), and are contained in an indolopyrrolocarbazole derivative (I) which is the final end product.

The present inventors continued studying a process for producing an indolopyrrolocarbazole derivative (I) or a pharmaceutically acceptable salt thereof and, as a result, found out the following points (a) to (d), resulting in completion of the present invention relating to a process for producing an indolopyrrolocarbazole derivative (I) or a pharmaceutically acceptable salt thereof which is excellent as an industrial production process.
(a) The contents of impurities contained in the compound (I) or a salt thereof as the final end product can be reduced.
(b) Yield of a compound (IV') or a salt thereof can be increased by improving a step of producing a compound (IV') or a salt thereof from a compound (V') or a salt thereof.
(c) The contents of impurities contained in a compound (IV') or a salt thereof can be reduced by improving a step of producing a compound (IV') or a salt thereof from a compound (V') or a salt thereof.

Since the impurities are not removed in the later step, and are mixed into an indolopyrrolocarbazole derivative (I) which is the final end product, or a pharmaceutically acceptable salt thereof, removal of the impurities is very important for industrial production of said final end product of high quality.
(d) Yield and purity of a compound (V') or a salt thereof can be improved by isolating the compound (V') as a crystalline solvate (it is generally thought that a solvate has better crystallizability as compared with a free-type compound) in the process for producing a compound (V') or a salt thereof.

That is, the present invention relates to the following (1) to (14).
(1) A process for producing an indolopyrrolocarbazole derivative represented by the formula (I): 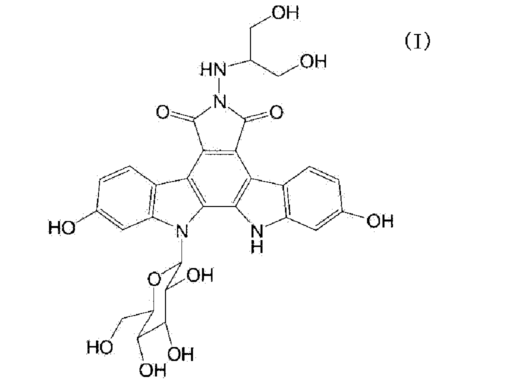 or a pharmaceutically acceptable salt thereof, which comprises:
   (i) the step of treating a compound represented by the formula (V): 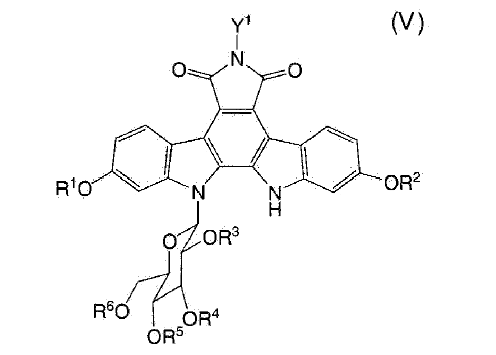 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group, or a solvate thereof or a salt thereof, with a base in an inert solvent, followed by treatment with an acid;
   (ii) the step of further treating the resulting reaction solution with a base in an inert solvent and subsequently with an acid to obtain a compound represented by the formula (IV): 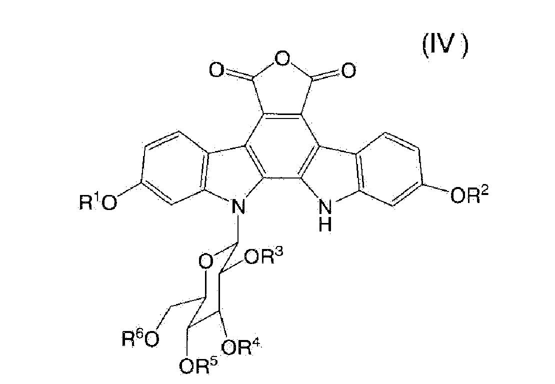 wherein R¹, R², R³, R⁴, R⁵ and R⁶ each independently represent a hydroxy protecting group, or salt thereof;
   (iii) the step of reacting the resulting compound of the formula (IV) with hydrazine diol represented by the formula (III): 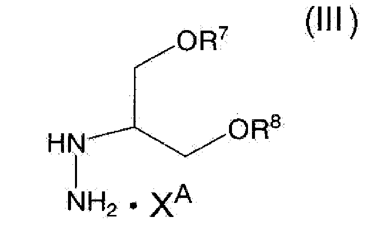 wherein X^{A} represents no existence of any molecule or an acid molecule; and R⁷ and R⁸ each independently represent a hydrogen atom or a hydroxy protecting group, or an acid addition salt thereof in the presence of an acid scavenger to obtain a compound represented by the formula (II): 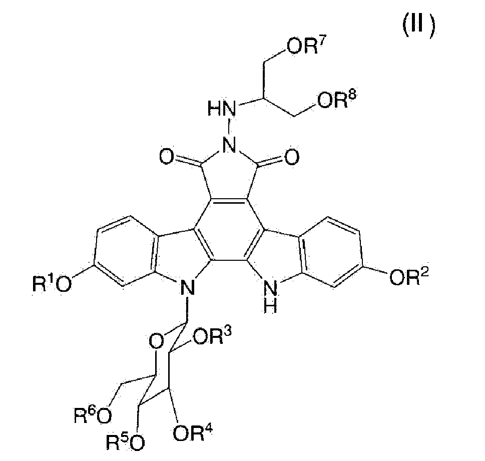 wherein R¹, R², R³, R⁴, R⁵ and R⁶ each independently represent a hydroxy protecting group; and R⁷ and R⁸ each independently represent a hydrogen atom or a hydroxy protecting group, or a salt thereof; and
   (iv) the step of removing the protecting groups from the resulting compound of the formula (II).
(2) The process according to the above (1), wherein a solvate or a salt of the compound represented by the formula (V) is a compound represented by the formula (VI): 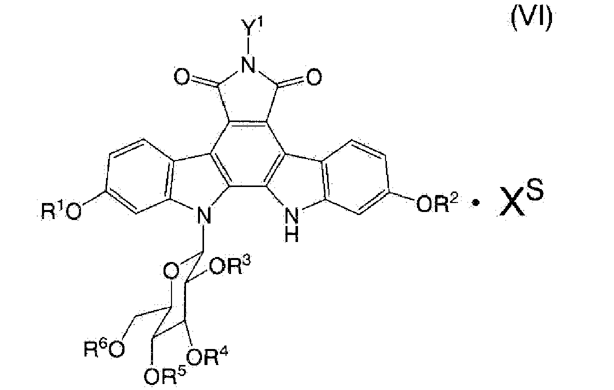 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently have the same meaning as defined below, or a salt thereof, which is obtained by carrying out the following steps (i) and (ii):
   (i) the step of reacting a compound represented by the formula (VIII): 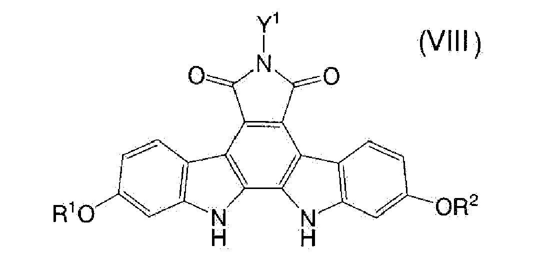 wherein Y¹ is defined above, R¹ and R² each independently represent a hydroxy protecting group, or a salt thereof, with a compound represented by the formula (VII): 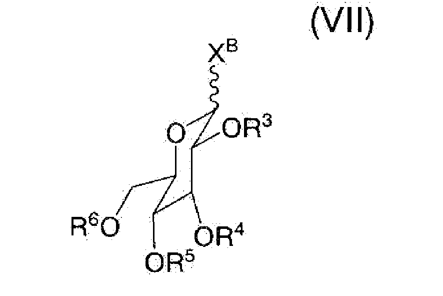 wherein X^{B} represents a halogen atom, and R³, R⁴, R⁵ and R⁶ each independently have the same meaning as defined above, in the presence of a base and a phase transfer catalyst; and
   (ii) the step of crystallizing the reaction product obtained in the above step as a solvate or a salt thereof.
(3) The process according to the above (1), wherein a solvate of the compound represented by the formula (V) is a compound represented by the formula (VI): 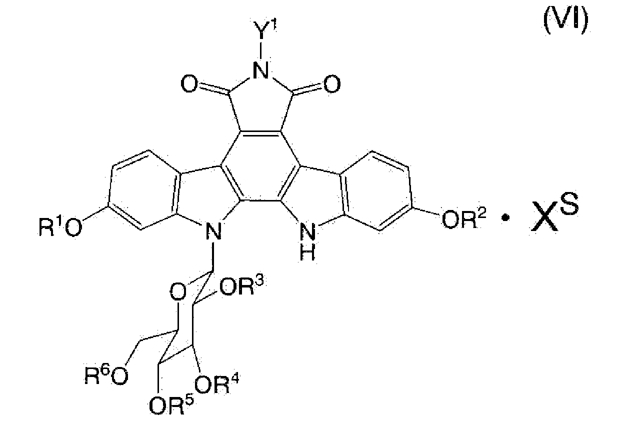 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.
(4) The process according to any one of the above (1) to (3), wherein R¹, R², R³, R⁴, R⁵, R⁶, R⁷ and R⁸ each represent a benzyl group.
(5) The process according to any one of the above (1) to (4), wherein X^{A} is 1/2 oxalic acid.
(6) The process according to any one of the above (1) to (5), wherein Y¹ is a methyl group.
(7) A solvate compound represented by the formula (VI): 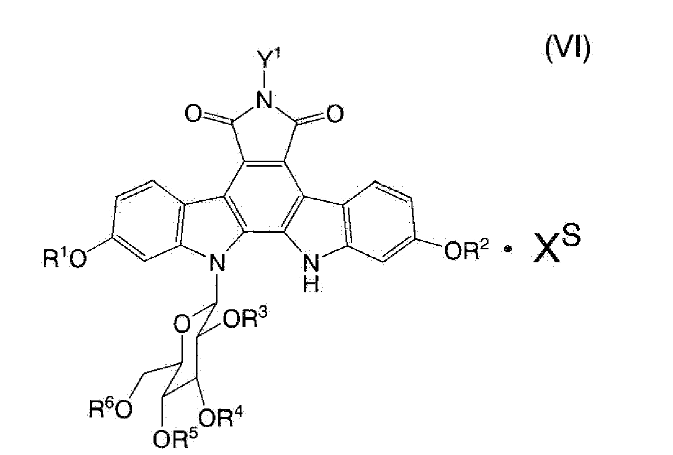 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵, and R⁶ each independently represent a hydroxy protecting group, or a salt thereof.
(8) The compound or a salt thereof according to the above (7), wherein the solvate compound represented by the formula (VI) is a compound represented by the formula (VIa): 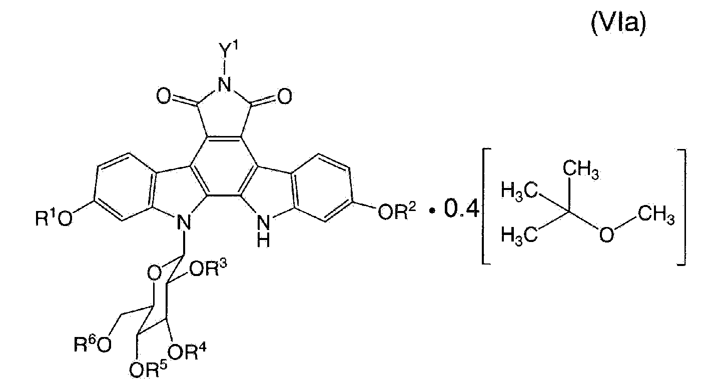 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.
(9) The compound or a salt thereof according to the above (7) or (8), wherein Y¹ is a methyl group, and R¹, R², R³, R⁴, R⁵ and R⁶ each represent a benzyl group.
(10) A process for producing a solvate compound represented by the formula (VI): 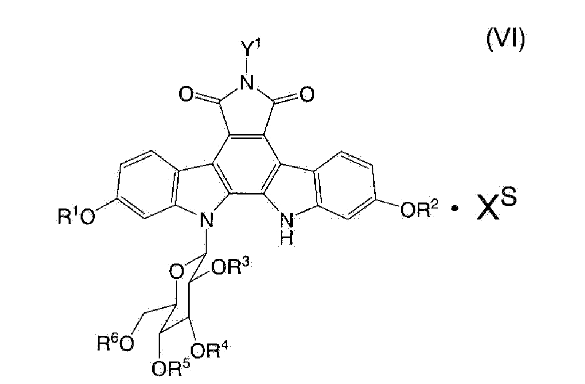 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group, or a salt thereof, which comprises:
   (i) the step of reacting a compound represented by the formula (VIII): 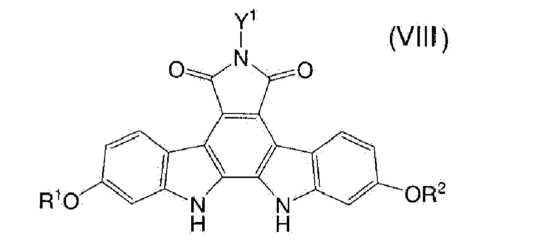 wherein Y¹ represents a hydrogen atom, a C₁-C₄ alkyl group, a phenyl group, a benzyloxymethyl group or an aralkyl group; and R¹ and R² each independently represent a hydroxy protecting group, or a salt thereof, with a compound represented by the formula (VII): 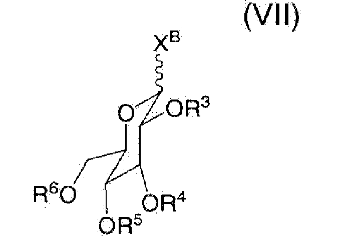 wherein X^{B} represents a halogen atom; and R³, R⁴, R⁵ and R⁶ each independently represent a hydroxy protecting group in the presence of a base and a phase transfer catalyst; and
   (ii) the step of crystallizing the reaction product obtained in the above step as a solvate or a salt thereof.
(11) The process according to the above (10), wherein the solvate compound represented by the formula (VI) is a compound represented by the formula (VIa): 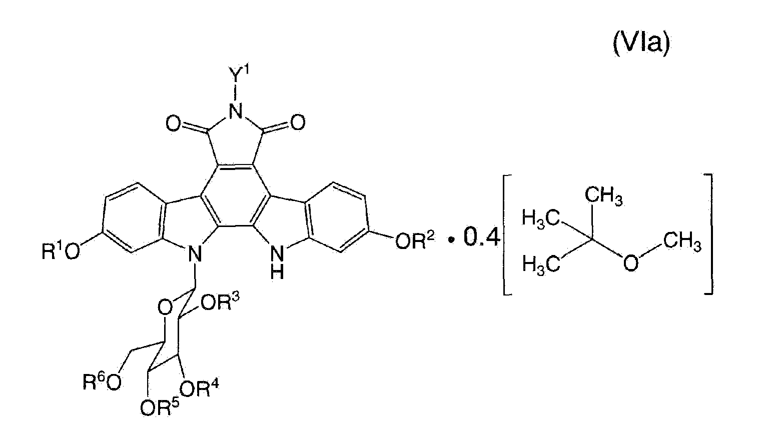 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.
(12) The process according the above (10) or (11), wherein Y¹ is a methyl group, and R¹, R², R³, R⁴, R⁵ and R⁶ each represent a benzyl group.
(13) A process for producing a compound represented by the formula (IV): 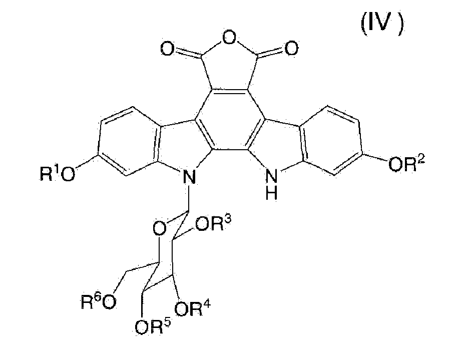 wherein R¹, R², R³, R⁴, R⁵ and R⁶ each independently represent a hydroxy protecting group, or a salt thereof, which comprises:
   (i) the step of treating a compound represented by the formula (V): 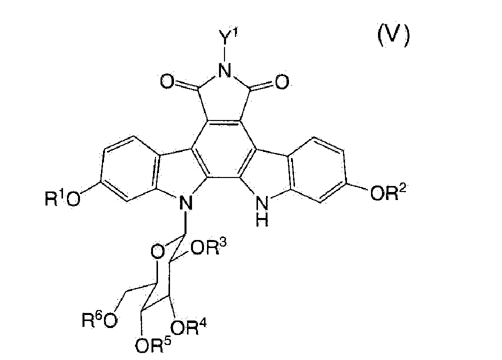 wherein Y¹ represents a hydrogen atom, a C₁-C₄ alkyl group, a phenyl group, a benzyloxymethyl group or an aralkyl group; and R¹, R², R³, R⁴, R⁵, and R⁶ each independently represent a hydroxy protecting group, or a salt thereof with a base in an inert solvent, followed by treatment with an acid; and
   (ii) the step of treating the reaction solution obtained in the above step with a base in an inert solvent and subsequently with an acid.
(14) The process according to the above (13), wherein R¹, R², R³, R⁴, R⁵ and R⁶ each represent a benzyl group.

According to the present process, improvement in yield and purity of a compound (V), a salt thereof or a solvate thereof, and improvement in yield and purity of a compound (IV) or a salt thereof can be achieved and, as a result, an indolopyrrolocarbazole derivative (I) useful as an anti-cancer agent in the pharmaceutical field, or a pharmaceutically acceptable salt thereof can be industrially produced more efficiently.

### BEST MODE FOR CARRYING OUT THE INVENTION

The present invention will be specifically explained in detail.

Terms used herein will be explained below.

The "C₁-C₄ alkyl group" means a straight or branched alkyl group having 1 to 4 carbon atoms, for example, a straight or branched alkyl group such as methyl, ethyl, propyl, isopropyl, butyl, isobutyl, t-butyl or the like, preferably methyl, ethyl, propyl, isopropyl, butyl or the like, more preferably methyl, ethyl, propyl or butyl.

The "aralkyl group" means the aforementioned "C₁-C₄ alkyl group" substituted with an aryl group such as phenyl, naphthyl or the like, for example, a C₇-C₁₂ aralkyl group such as benzyl, 1-naphthyl methyl, 2-naphthyl methyl or the like, preferably benzyl.

The "acid molecule" means a protonic acid such as hydrochloric acid, sulfuric acid, nitric acid, acetic acid, methylsulfonic acid, p-toluenesulfonic acid, oxalic acid, citric acid, propionic acid or the like, preferably 1/2 oxalic acid.

The "acid" means a protonic acid such as hydrochloric acid, sulfuric acid, nitric acid, acetic acid, methylsulfonic acid, p-toluenesulfonic acid, oxalic acid, citric acid, propionic acid or the like, preferably citric acid.

Examples of the "hydroxy protecting group" include a protecting group for a hydroxy group such as benzyl, trityl, p-methoxybenzyl, benzyloxy methyl group or the like, preferably benzyl.

Examples of the "base" include a base such as sodium hydroxide, lithium hydroxide, cesium hydroxide, barium hydroxide, magnesium hydroxide, potassium hydroxide, potassium methoxide, sodium methoxide, sodium t-butoxide, potassium t-butoxide or the like, among which sodium hydroxide, potassium hydroxide, sodium methoxide or the like is preferable.

The "solvate" means, in the case where the final end product is crystallized in a production process, a crystal form which is formed by incorporating solvent molecules (in particular inert organic solvent molecule) used in the production process into its crystal lattice. Examples of the "solvent molecule" include hydrocarbon such as toluene, xylene, heptane, hexane or the like; ether such as t-butyl methyl ether, tetrahydrofuran or the like; halogenated hydrocarbon such as methylene chloride, carbon tetrachloride, chloroform, dichlorobenzene or the like; ketone such as methyl isobutyl ketone, acetone or the like; and a nonionic solvent such as N,N-dimethylformamide, 1-methyl-2-pyrrolidinone or the like, preferably t-butyl methyl ether.

The "salt" may be any salt as far as it is a salt with an acid which is a pharmaceutically acceptable salt. Examples thereof include a salt with an inorganic acid such as hydrochloric acid, sulfuric acid or the like, and a salt with an organic acid such as acetic acid, methylsulfonic acid, p-toluenesulfonic acid or the like. Preferable examples of the pharmaceutically acceptable salt include a salt with hydrochloric acid, sulfuric acid, citric acid, acetic acid or the like.

Hereinafter, a production process of the present invention will be specifically explained.

First, a step of reacting a compound represented by the formula (VIII); 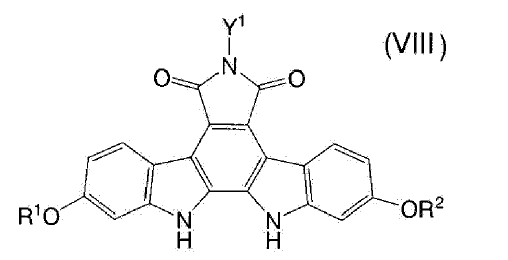 wherein Y¹ represents a hydrogen atom, a C₁-C₄ alkyl group, a phenyl group, a benzyloxymethyl group or an aralkyl group; and R¹ and R² each independently represent a hydroxy protecting group, or a salt thereof with a compound represented by the formula (VII): 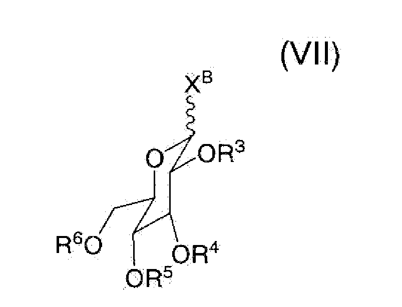 wherein X^{B} represents a halogen atom; and R³, R⁴, R⁵ and R⁶ each independently represent a hydroxy protecting group, or a salt thereof in the presence of a base and a phase transfer catalyst to produce a compound represented by the formula (V): 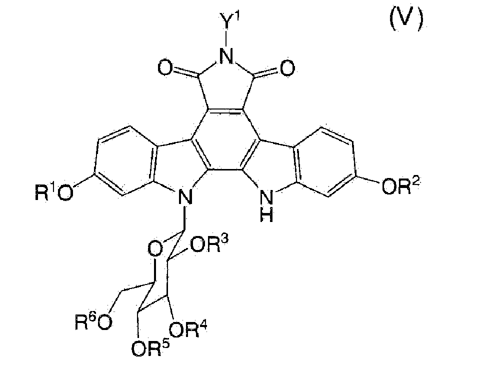 wherein Y¹ represents a hydrogen atom, a C₁-C₄ alkyl group, a phenyl group, a benzyloxymethyl group an aralkyl group; and R¹, R², R³, R⁴, R⁵ and R⁶ each independently represent a hydroxy protecting group, or a salt thereof can be performed by reacting a glucose derivative represented by the formula (VIIa): 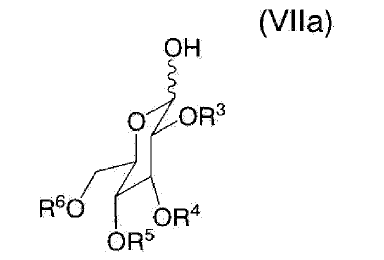 wherein R², R³, R⁴, R⁵ and R⁶ each independently represent a hydroxy protecting group with a halogenating agent such as acid halide, sulfonyl chloride, iodine-triphenylphosphine, iodine-triphenylphosphine or the like at about -50°C to about 200°C, preferably at about -10°C to 30°C in an inert solvent, to produce an activated glucose derivative represented by the formula (VII): 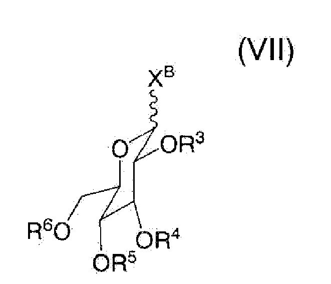 wherein R³, R⁴, R⁵ and R⁶ each independently represent a hydroxy protecting group; and X^{B} represents a halogen atom such as chlorine, iodine or the like, and then coupling the resulting activated glucose derivative of the formula (VII) with a compound presented by the general formula (VIII): 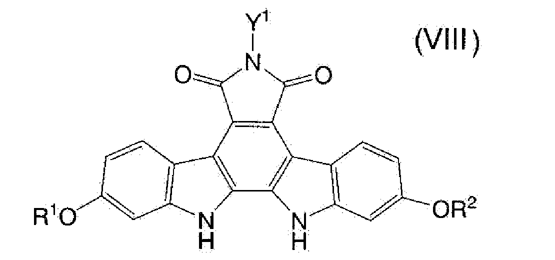 wherein R¹, R² and Y¹ each have the same meaning as defined above,
or a salt thereof at about -50°C to 200°C, preferably at about 0°C to 40°C using a base and a phase transfer catalyst, preferably using a biphasic system comprising a base in an aqueous solvent and a phase transfer catalyst in an inert organic solvent.

Examples of the acid halide used in the step of producing an activated glucose derivative include SOCl₂, POCl₃, SOBr₃, POBr₃, PBr₃, oxalic acid chloride or the like, preferably SOCl₂ or oxalic acid chloride, most preferably SOCl₂.

Examples of the inert solvent used in the step of producing an activated glucose derivative include hydrocarbon such as toluene, xylene, heptane, hexane or the like; nitrile such as acetonitrile or the like; ether such as t-butyl methyl ether, tetrahydrofuran or the like; halogenated hydrocarbon such as methylene chloride, carbon tetrachloride, chloroform, dichlorobenzene or the like; and ketone such as methyl isobutyl ketone, acetone or the like, preferably t-butyl methyl ether.

As the glucose derivative of the formula (VIIa), a commercially available product may be utilized.

An example of the aqueous solvent used in the step of coupling an activated glucose derivative and a compound of the formula (VIII) includes water.

Examples of the base used in the step of coupling an activated glucose derivative and a compound of the formula (VIII) include alkali hydroxide such as lithium hydroxide, sodium hydroxide, potassium hydroxide, cesium hydroxide or the like, preferably sodium hydroxide and potassium hydroxide. The concentration of the base in an aqueous solvent is about 5 wt.% to about 95 wt.%, preferably 45 wt.% to about 50 wt.%.

Examples of the inert organic solvent used in the step of coupling an activated glucose derivative and a compound of the formula (VIII) include hydrocarbon such as toluene, xylene, heptane, hexane or the like; nitrile such as acetonitrile or the like; ether such as t-butyl methyl ether, tetrahydrofuran or the like; halogenated hydrocarbon such as methylene chloride, carbon tetrachloride, chloroform, dichlorobenzene or the like; ketone such as methyl isobutyl ketone, acetone or the like; and a nonionic solvent such as N,N-dimethylformamide, 1-methyl-2-pyrrolidinone or the like, preferably t-butyl methyl ether.

Examples of the phase transfer catalyst used in the step of coupling an activated glucose derivative and a compound of the formula (VIII) include a compound represented by the formula (IX): 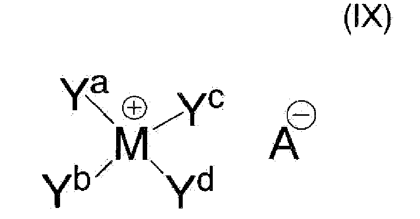 wherein Y^{a}, Y^{b}, Y^{c} and Y^{d} each independently represent a hydrogen atom, a benzyl group or a hydrocarbon group having 1 to 18 carbon atoms; M represents a nitrogen atom or a phosphorus atom; and A represents a hydroxy group, a fluorine atom, a bromine atom, a chlorine atom, an iodine atom, a cyano group, HSO₄, CH₃SO₃, an acetyl group or PhCH₂COO, and tris(2-(2-methoxyethoxy)ethyl)amine, among which tricaprylmethylammonium chloride, tris(2-(2-methoxyethoxy)ethyl)amine, benzyltriethylammonium chloride and tributylammonium hydrogen sulfate are preferable, and tricaprylmethylammonium chloride is more preferable.

The step of crystallizing the reaction product (V) obtained in the step of introducing a glucose derivative (VII) or a salt thereof as a solvate, to produce a compound represented by the formula (VI): 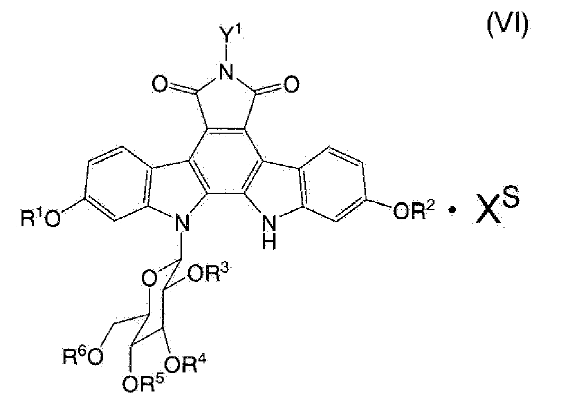 wherein X^{S} represents a solvent molecule; and Y¹, R¹, R², R³, R⁴, R⁵ and R⁶ each have the same meaning as defined above, or a salt thereof can be performed by separating an organic layer from the reaction solution obtained in the step of coupling an activated glucose derivative and a compound of the formula (VIII), washing the organic layer with water two or three times, concentrating the layer to reduce the fluid volume by half at 0°C to 50°C, preferably 20°C to 30°C under reduced pressure, dropwise adding, for example, 6N hydrochloric acid/methanol to the resulting concentrated solution at room temperature over 2 hours to 5 hours, preferably 3 hours to 4 hours to adjust the pH at 2.0 to 4.0, preferably at 2.5 to pH 3.5, performing aging at room temperature for 10 hours to 30 hours, preferably for 15 hours to 25 hours, filtering the resulting suspension, and drying the resulting crystal at 0°C to 30°C, preferably 10°C to 25°C under reduced pressure.

The next step of treating the compound of the formula (VI): 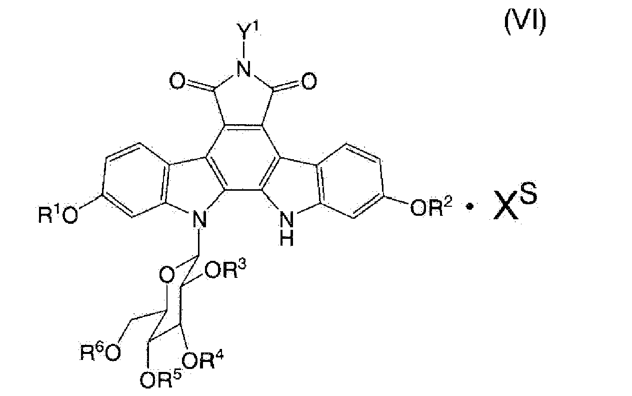 wherein X^{S}, Y¹, R¹, R², R³, R⁴, R⁵ and R⁶ each have the same meaning as defined above, or a salt thereof obtained in the previous step, or a compound represented by the formula (V): 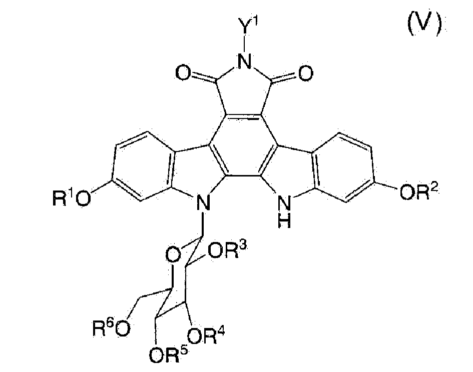 wherein Y¹ represents a hydrogen atom, a C₁-C₄ alkyl group, a phenyl group, a benzyloxymehtyl group or an aralkyl group; and R¹, R², R³, R⁴, R⁵ and R⁶ each independently represent a hydroxy protecting group, or a salt thereof with a base in an inert solvent, followed by treatment with an acid, and a step of further treating the resulting reaction solution with a base in an inert solvent, followed by treatment with an acid to produce a compound represented by the formula (IV): 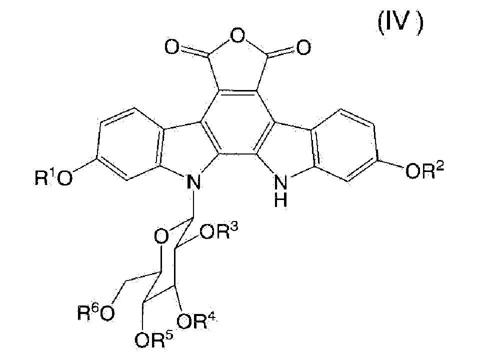 wherein R¹, R², R³, R⁴, R⁵ and R⁶ each have the same meaning as defined above, can be usually performed by repeating twice the steps of treatment with a base in an inert solvent having no adverse influence on reactions using 50 to 100 moles, preferably 50 to 70 moles of the base relative to 1 mole of the compound of the formula (V) or formula (VI), or a salt thereof, and treatment with an acid, under the same reaction conditions.

The reaction conditions for the first and second operations of the above base treatment and acid treatment may be the same or different, preferably the same. Further, a series of the above steps of base treatment in an inert solvent and subsequent acid treatment may be repeated three or more times.

The present inventors found that, by twice performing a series of steps of base treatment in an inert solvent and subsequent acid treatment as mentioned above, production of a byproduct is unexpectedly decreased and, as a result, a compound (IV) as the end product having high purity can be produced at a high yield.

Examples of the inert solvent having no adverse influence on reactions used in the step of producing a compound (IV) from a compound (V) or a compound (VI) include an alcohol such as methanol, ethanol, isopropanol, t-butanol or the like, dimethyl sulfoxide, toluene and a mixed solvent thereof, among which methanol, ethanol, toluene, a mixed solvent thereof or the like is particularly preferable.

Examples of the base used in the step of producing a compound (IV) from a compound (V) or a compound (VI) include a base such as sodium hydroxide, potassium hydroxide, potassium methoxide, sodium methoxide, sodium t-butoxide, potassium t-butoxide or the like, preferably sodium hydroxide, potassium hydroxide, sodium methoxide or the like.

The acid used in the step of producing a compound (IV) from a compound (V) or a compound (VI) means a protonic acid such as hydrochloric acid, sulfuric acid, nitric acid, acetic acid, methylsulfonic acid, p-toluenesulfonic acid, oxalic acid, citric acid, propionic acid or the like, preferably citric acid.

For the base treatment in the step of producing a compound (IV) from a compound (V) or a compound (VI), reaction temperature is usually about 0°C to 40°C, preferably at about 20°C to 30°C, and reaction time is usually 1 hour to 1 day, preferably 1 hour to 10 hours.

For the acid treatment in the step of producing a compound (IV) from a compound (V) or a compound (VI), reaction temperature is usually about -10°C to 70°C, preferably at about 20°C to 50°C, and reaction time is usually 1 hour to 1 day, preferably 1 hour to 10 hours.

In addition, a step of reacting a compound represented by the formula (IV): 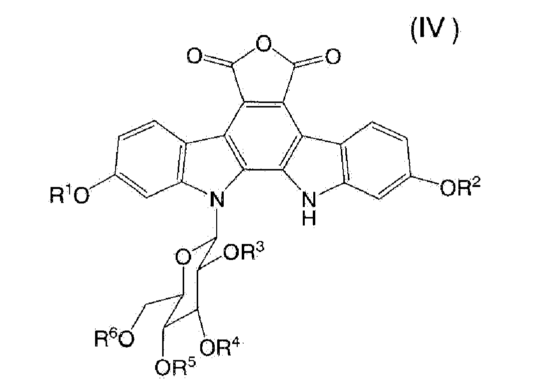 wherein R¹, R², R³, R⁴, R⁵ and R⁶ each independently represent a hydroxy protecting group, or a salt thereof obtained in the previous step with an acid addition salt of hydrazine diol represented by the formula (III): 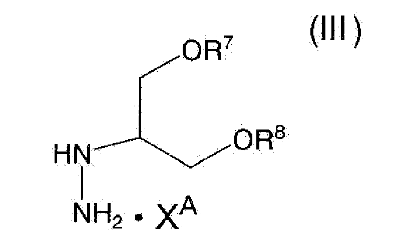 wherein X^{A} is absent or represents an acid molecule; and R⁷ and R⁸ each independently represent a hydrogen atom or a hydroxy protecting group in the presence of an acid scavenger to produce a compound represented by the formula (II): 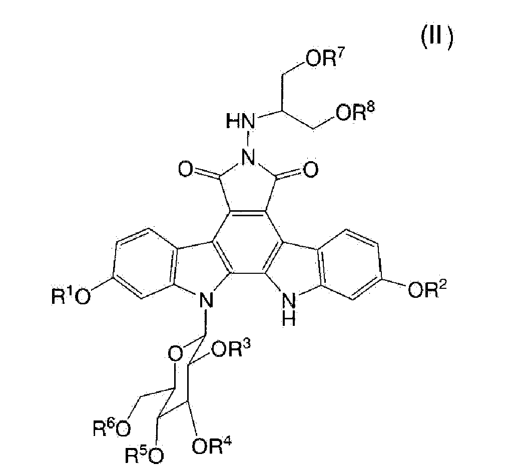 wherein R¹, R², R³, R⁴, R⁵, R⁶, R⁷ and R⁸ each have the same meaning as defined above, can be performed as follows: that is, usually, the step can be performed in the presence of an acid scavenger in an inert solvent having no adverse influence on reactions using equivalent mole to 1.5 moles, preferably 1.2 to 1.3 moles of the compound of the formula (III) relative to 1 mole of the compound of the formula (IV).

Examples of the inert solvent having no adverse influence on reactions used in the step of producing a compound (II) from a compound (IV) include N,N-dimethylformamide, N,N-dimethylacetamide, tetrahydrofuran, dimethyl sulfoxide and N-methylpyrrolidone, or a mixed solvent thereof, among which N,N-dimethylformamide, N,N-dimethylacetamide, N-methylpyrrolidone or the like is preferable.

In the step of producing a compound (II) from a compound (IV), reaction temperature is usually room temperature to about 60°C, preferably at about 30°C to 50°C, and reaction time is usually 1 hour to 1 day, preferably 1 hour to 3 hours.

Examples of the acid scavenger used in the step of producing a compound (II) from a compound (IV) include triethylamine and 4-dimethylaminopyridine, preferably triethylamine.

A step of removing protecting group(s) from a compound represented by the formula (II): 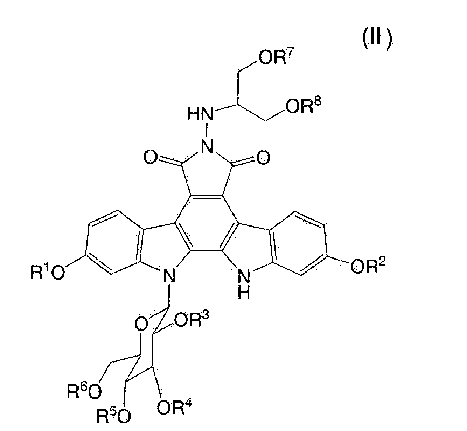 wherein R¹, R², R³, R⁴, R⁵, R⁶, R⁷ and R⁸ each have the same meaning as defined above obtained in the previous step to produce an indolopyrrolocarbazole derivative represented by the formula (I): 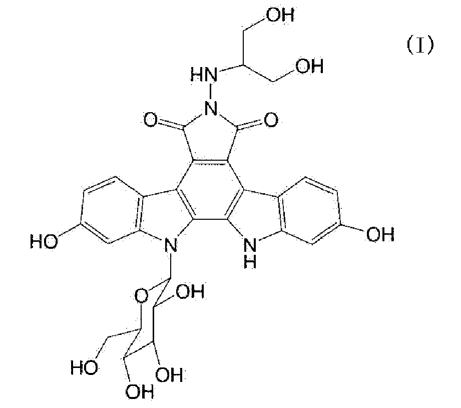 or a pharmaceutically acceptable salt thereof can be performed, for example, by catalytic reduction reaction in the presence of, for example, a catalyst used in catalytic reduction reaction such as palladium-carbon or the like under, for example, hydrogen gas atmosphere.

Hydrogen gas pressure in the catalytic reduction reaction used in the step of producing a compound (I) from a compound (II) is usually preferably a normal pressure to 2 atom, and the amount of catalyst to be used in the catalytic reduction reaction is usually 1/100- to 1-fold, preferably 1/100- to 1/10-fold relative to the weight of compound (II) as raw material.

Examples of the reaction solvent used in the step of producing a compound (I) from a compound (II) include a mixed solvent of an alcohol solvent such as methanol, ethanol, isopropanol, butanol or the like and tetrahydrofuran, preferably a mixed solvent of isopropanol and tetrahydrofuran (60/40).

In the step of producing a compound (I) from a compound (II), reaction temperature is usually about -30°C to 60°C, preferably at about 0°C to 50°C, and reaction time is usually 10 minutes to 7 days.

Objective compounds obtained by the aforementioned respective production steps can be purified and isolated following the known per se method such as a conventional separation and purification method (e.g. solvent extraction, recrystallization/reprecipitation) alone or in combination.

An indolopyrrolocarbazole derivative (I) or a pharmaceutically acceptable salt thereof obtained by the aforementioned steps contains little, if any, byproduct.

### EXAMPLES

The present invention will be further explained specifically by way of Examples and Comparative Examples, but the present invention is not limited by them.

### Example 1

Production of 12,13-dihydro-2,10-dibenzyloxy-13-(β-D-2,3,4,6-tetra-O-benzylglucopyranosyl)-5H-indolo[2,3-a]pyrrolo[3,4-c]carbazole-6-methyl-5,7(6H)-dione 0.4 t-butyl methyl etherate

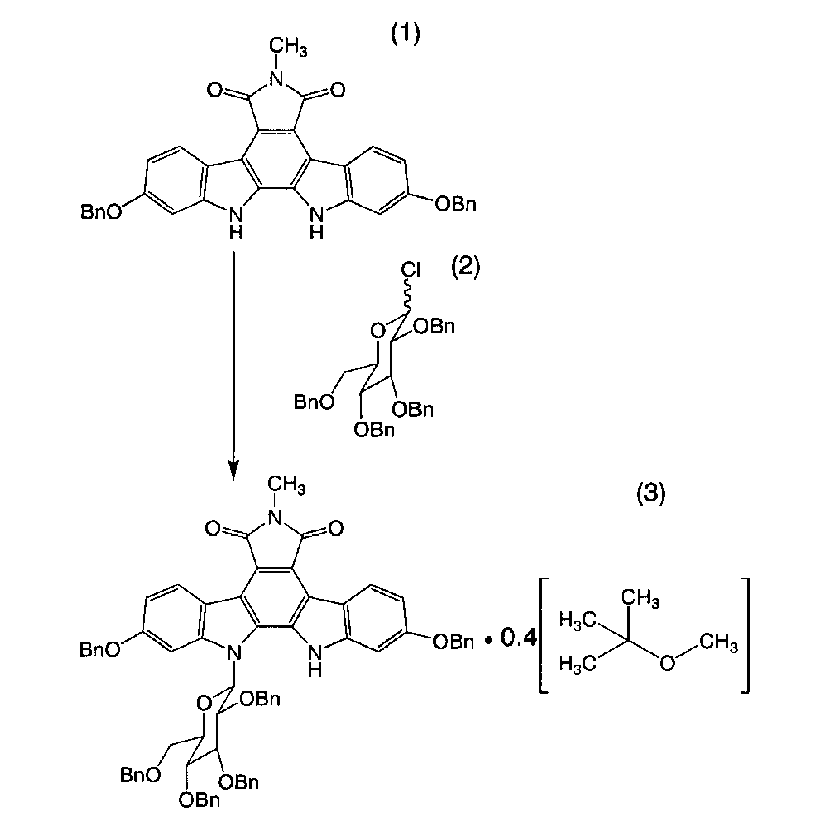

In the above reaction equation, Bn is a benzyl group. t-butyl methyl ether (61 mL) and the compound (1) (10.2 g, 18.5 mmol, 1 equivalent) were placed in a 1L three-neck flask equipped with a mechanical stirrer, a thermometer and a N₂ introducing tube. The inner wall of the flask was washed using t-butyl methyl ether (31 mL). This suspension was stirred at 20 to 25°C for 10 minutes, and a solution of 1-chloro-2,3,4,6-O-tetrabenzyl-D-glucopyranose in t-butyl methyl ether (prepared according to the disclosure of WO 02/36601)(70 mL) was added. T-butyl methyl ether (14 mL) was used to wash the flask for transferring the solution. This mixture (yellow suspension) was stirred at room temperature for 30 minutes, and a 48% by weight aqueous KOH solution (75 g (containing 36 g as potassium hydroxide), 640 mmol, 35 equivalents) was added at room temperature over 5 minutes to obtain a two-layered mixture. The mixture was stirred at room temperature for 30 minutes, and a solution of Aliquat 336 [trade mark of tricaprylmethylammonium chloride (Aldrich Chemical Co., Inc.)] (10.5 g, 25.9 mmol, 1.4 equivalents) in t-butyl methyl ether (51 mL) was added over 15seconds. The resulting dark red two-layered mixture was stirred at room temperature (20 to 25°C) for 4 hours.

Stirring was stopped, the aqueous layer was removed, and the organic layer (dark red solution) was washed with water (2 × 50 mL). A water content of the resulting organic layer was measured (t-butyl methyl ether layer: 253 mL, KF 1.78%).

This dark red solution was allowed to stand at room temperature over night (12 hours), and concentrated to 132.5 mL at 30°C (bath temperature) under reduced pressure. To this concentrated solution was added dropwise a 6N-hydrochloric acid (1.23 mL, 7.38 mmol)/methanol (10.2 mL) mixed solution at room temperature over 3 minutes, the mixture was stirred for 5 minutes, a seed crystal (100 mg) of an end product was added as a suspension in t-butyl methyl ether (1.0 mL), and this was stirred at room temperature for 1 hour. Crystal growth was confirmed, and a suspension was obtained. Subsequently, a 6N hydrochloric acid (2.17 mL, 13.0 mmol)/methanol (140 mL) mixed solution was added over 2.5 hours until the pH of the mixture became to be 2.5 to 3.5. Usually, about 120 mL of the mixed solution is necessary. After addition of such mixed solution, the mixture was aged at room temperature for 21 hours, and crystals were filtered, and washed with t-butyl methyl ether-methanol (1:4, v/v)(2 × 20 mL), and dried at 25°C and about 2.5 mmHg for 11.5 hours to obtain 12,13-dihydro-2,10-dibenzyloxy-13-(β-D-2,3,4,6-tetra-O-benzylglucopyranosyl)-5H-indolo[2,3-a]pyrrolo[3,4-c]carbazole-6-methyl-5,7(6H)-dione•0.4 t-butyl methyl etherate (12.704 g, yield: 93%) as yellow crystals.
¹H-NMR (500.13 MHz, CDCl₃) δ(ppm): 10.64(1H, s), 9.22-9.24(1H, m), 9.11-9.13(1H, m), 7.47-7.48(2H, m), 7.42-7.44(2H, m), 7.38-7.41(2H, m), 7.32-7.37(6H, m), 7.24-7.30(8H, m), 7.18-7.21(5H, m), 7.14- 7.16(2H, m), 7.09-7.11(2H, m), 6.99-7.02(1H, m), 6.87-6.88(2H, m), 6.20(2H, dd, J=1.1, 8.2), 5.83(1H, d, J=9.0), 5.17(1H, d, J = 11.4 Hz), 5.16, 5.19(1H each, ABd, J=11.4Hz), 5.08(1H, d, J=11.7Hz), 4.97(1H, d, J=10.6 Hz), 4.85, 4.88(1H each, ABd, J=10.9 Hz), 4.56(1H, d, J= 13.1Hz), 4.32(1H, t, J = 9.5 Hz), 4.02(1H, t, J=9.1 Hz), 4.00(1H, d, J=3.0Hz), 3.91(2H, m), 3.86(1H, d, J=5.4Hz), 3.79(1H, dd, J=2.5, 10.2 Hz), 3.31(3H, s), 3.21(3Hx0.4, s), 3.01(1H, d, J=9.8Hz), 1.19(9Hx0.4, s).
¹³C-NMR(125.77MHz,CDCl₃) δ (ppm): 23.74,26.99,49.45, 66.76, 70.40,70.67,72.79,73.96,74.88,75.42,75.88,75.99,77.36,80.37,84.77,85.71,96.35,96.68,116.72,116.87,11 8.27,118.95,119.32,120.12,126.48,126.74,127.34,127.60,127.70,127.76,127.84,127.93,127.97,128.03,12 8.09,128.13,128.19,128.26,128.35,128.48,128.56,128.62,128.73,130.50,136.11,136.70,136.88,136.95,13 7.66,137.97,142.93,143.18,159.3 6,170.25.

### Thermogravimetric analysis (TG method)

### Measurement conditions:

Nitrogen gas flow rate: 100 mL/min
Temperature raising rate: raising to 500°C at 10°C/min
Decrease in amount %: 3.003 % (22.93°C to 105.18°C)
X-Ray Powder diffraction data:

| 2θ (°) | Relative intensity (%) |
|---|---|
| 4.5 | 29.8 |
| 5.1 | 100.0 |
| 5.8 | 84.7 |
| 5.9 | 59.9 |
| 6.9 | 68.3 |
| 7.0 | 58.2 |
| 7.7 | 30.5 |
| 8.3 | 24.1 |
| 10.2 | 9.9 |
| 10.7 | 7.2 |
| 11.4 | 9.4 |
| 12.0 | 7.1 |
| 12.5 | 6.7 |
| 13.3 | 8.9 |
| 15.2 | 13.4 |
| 15.9 | 31.3 |
| 16.9 | 46.7 |
| 17.2 | 57.3 |
| 18.1 | 41.3 |
| 18.8 | 56.2 |
| 19.9 | 41.3 |
| 20.5 | 42.2 |
| 21.0 | 34.2 |
| 21.9 | 28.9 |
| 22.8 | 32.6 |
| 25.2 | 17.4 |
| 26.6 | 15.2 |
| 27.9 | 8.4 |
| 29. 3 | 4.7 |

### Measurement conditions of X-ray powder diffraction data:

Measuring apparatus: X-ray powder diffraction apparatus X'Pert Pro (manufactured by PANanalytical)

| | |
|---|---|
| Scan axis | Gonio |
| Start Position | 4.0170 [°2θ] |
| End position | 39.9550 [°2θ] |
| Step Size | 0.0170 [°2θ] |
| Scan step time: | 20.9550 [sec] |
| Kind of scan | Continuous measurement |
| PSD mode: | Scanning |
| PSD distance: | 2.13 [°2θ] |
| Offset: | 0.00 [°2θ] |
| Divergence slit(DS)type: | Fixation |
| Divergence slit(DS) size: | 0.2500 [°] |
| Irradiation width: | 10.00 [mm] |
| Sample width: | 10.00 [mm] |
| Measuring temperature: | 25.00 [°C] |
| Target: | Cu |
| X-ray output setting: | 45 kV, 40 mA |
| Goniometer radius: | 240.00 [mm] |
| Distance between forcus-DS: | 100.00 [mm] |
| Incident side monochrometer: | Absence |
| Spinner: | Presence |

### Comparative Example 1

### Step 1:

2,3,4,6-O-tetrabenzyl-D-glucopyranose (17.41 kg, 27.2 moles) was mixed with dimethylformamide (DMF, 58.8 L) at 20°C, and the mixture was cooled to -0.5°C. Thionyl chloride (3.71 kg, 31.2 moles) was slowly added over 15 minutes. The solution was warmed to about 25°C, and aged for 2 hours. Then, to the solution was added t-butyl methyl ether (66 L). The solution was cooled to 0°C, and 0.5N NaOH (210 L) was added. During this, the temperature did not exceed 5°C. The solution was warmed to 22°C, and stirred for 30 minutes. The aqueous layer was separated, and the organic layer was washed with water (2 × 38 L) and a 25% by weight aqueous sodium chloride solution (38 L). The solution was used in the next step without purification.

### Step 2:

The above compound (1) (10.0 kg, 18.13 moles) was suspended in t-butyl methyl ether (100 L), and stirred at 23°C for 15 minutes. Then, a solution of the compound produced in the step 1 was added, and a 48%(w/w) KOH aqueous solution (50 L) was added after 30 minutes. After further 10 minutes, 26% (w/w) Aliquat (registered trademark) 336 (10.26 kg in 129.6 kg MTBE) was added over 6 minutes. Aliquat (registered trademark) 336 is a trade name of tricaprylmethylammonium chloride sold from Aldrich Chemical Co., Inc. located in Wisconsin, Milwaukee. After the solution was aged at 23°C for 3 hours, the aqueous layer was separated. The organic layer was washed with water (50 L). The solution was cooled to 5°C, and 1N hydrochloric acid (20 kg) was added. The solution was warmed to 22°C, and 1N hydrochloric acid (2.5 kg) was further added. The solution was stirred for 30 minutes, and the aqueous layer was separated. Then, the organic layer was washed with a 5% by weight aqueous sodium chloride solution (50 L) and a 25% by weight aqueous sodium chloride solution (50 L). The solution was concentrated to 140 L under reduced pressure. The solution was adjusted to 23°C, and methanol (20 L) was added slowly. A seed crystal (5 g) of an end product was added, and this was stirred at 22°C for 2 hours. Crystal growth was confirmed, and a suspension was obtained. Subsequently, methanol (80 L) was added slowly over 1 hour. After stirred at room temperature overnight, crystals were filtered, washed with t-butyl methyl ether-methanol (1:4, v/v)(3 × 50 L), and dried at 25°C for 21 hours under reduced pressure to obtain 12,13-dihydro-2,10-dibenzyloxy-13-(β-D-2,3,4,6-tetra-O-benzylglucopyranosyl)-5H-indolo[2,3-a]pyrrolo[3,4-c]carbazole-6-methyl-5,7(6H)-dione (16.19 kg, yield: 83%) as yellow crystals.
Yield of end product:
In case of Example 1: 93%
In case of comparative Example 1: 83%

### Example 2

### Preparation of 12,13-dihydro-2,10-dibenzyloxy-13-(β-D- 2,3,4,6-tetra-O-benzylglucopyranosyl)-5H-indolo[2,3-a]carbazole-5,6-dicarboxylic acid anhydride

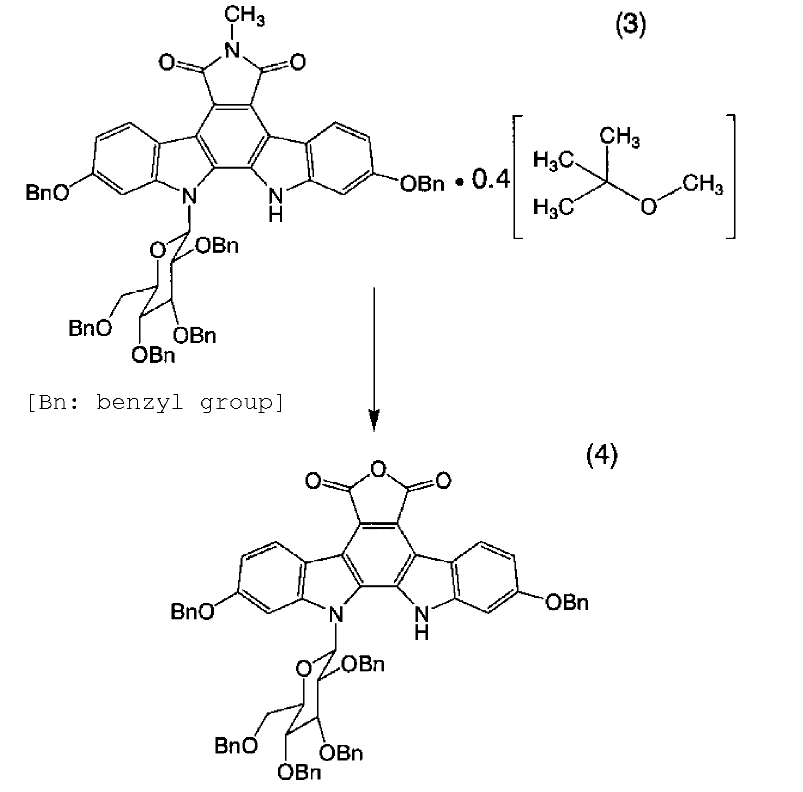

A stirrer and a thermometer were set on a 50 L-flask, and a 48% by weight aqueous potassium hydroxide solution (2.7 L) was placed therein. 12,13-dihydro-2,10-dibenzyloxy-13-(β-D-2,3,4,6-tetra-O-benzylglucopyranosyl)-5H-indolo[2,3-a]pyrrolo[3,4-c]carbazole-6-methyl-5,7(6H)-dione•0.4 t-butyl methyl etherate (1.5 kg, 1.35 mol) obtained in Example 1 was placed therein while stirring, and subsequently toluene (5.4 L) was placed therein, and the mixture was stirred at room temperature for 30 minutes. Ethanol (13.5 L) was added dropwise to the mixture at the same temperature over 30 minutes, and the solution was stirred at room temperature overnight. The resulting red brown solution was cooled to -5°C or lower, a 10% by weight aqueous citric acid solution (29 L) was added dropwise over 30 minutes to pH 6.3, and the mixture was stirred at room temperature for 2 hours. To this yellow solution was added methyl tert-butyl ether (12 L), and the solution was stirred for 10 minutes. The aqueous layer (lower layer) was separated, and the organic layer was successively washed with a 3% by weight sodium chloride solution (3 L × 2), and purified water (3 L). After removal of the solvent by evaporation under reduced pressure, toluene (5.4 L × 2) was added, and the solvent was removed by evaporation under reduced pressure, thereby substituting the solvent with toluene. A solution of the objective crude 12, 13-dihydro-2,10- dibenzyloxy-13-(β-D- 2,3,4,6-tetra-O-benzylglucopyranosyl)-5H-indolo[2,3-a]carbazole-5,6-dicarboxylic acid anhydride in toluene was obtained as a yellow oily residue. A 48% by weight aqueous potassium hydroxide solution (2.7 L) was added into the solution of the crude product in toluene. Ethanol (13.5 L) was added dropwise thereto at room temperature over 30 minutes while stirring, and the mixture was stirred at room temperature for 2 hours. The resulting red brown solution was cooled to -5°C or lower, and a 10% by weight aqueous citric acid solution (28.5 L) was added dropwise over 30 minutes to pH 6.3. The resulting yellow solution was warmed to 60°C, stirred at the same temperature for 2 hours, and cooled to room temperature, and then t-butyl methyl ether (12L) was added. After stirring for 10 minutes, the aqueous layer (lower layer) was separated, and the organic layer was successively washed with a 3% by weight aqueous sodium chloride solution (3 L × 2). The solvent was evaporated under reduced pressure, acetonitrile (15 L × 2) was added to the residue, and the solvent was evaporated under reduced pressure, thereby substituting the solvent with acetonitrile. Then acetonitrile (22.5 L) was further added, the resulting yellow solution was adjusted to room temperature, and methanol (7 L) was added thereto slowly. Subsequently, a seed crystal was added to the solution, and the mixture was stirred at the same temperature for 1 hour. Further, purified water (7.5 L) was added dropwise to the solution over 2 hours. After stirred for 4 hours, the suspension was filtered, and crystals were washed with a mixture of acetonitrile and methanol, and dried under reduced pressure to obtain the objective 12,13-dihydro-2,10- dibenzyloxy-13-(β-D-2,3,4,6-tetra-O-benzylglucopyranosyl)- 5H-indolo[2,3-a]carbazole-5,6-dicarboxylic acid anhydride as a yellow crystalline substance (1.3 kg; yield 92%).
¹H-NMR(270 MHz,CDCl₃), δ(ppm):10.79(1H,br.s), 9.04(1H,d,J = 9.2 Hz), 8.95(1H,d,J=9.6Hz), 7.26(32H,m), 6.17(2H,d,J=7.3Hz), 5.85 (1H,d,J= 8.2 Hz), 4.89(10H,m), 4.32(1H,t,J = 8.9 Hz), 3.96(6H,m), 3.13(1H,d,J=10.2Hz)

### Comparative Example 2

### Preparation of 12,13-dihydro-2,10-dibenzyloxy-13-(β-D-2,3,4,6-tetra-O-benzylglucopyranosyl)-5H-indolo[2,3- a]carbazole-5,6-dicarboxylic acid anhydride

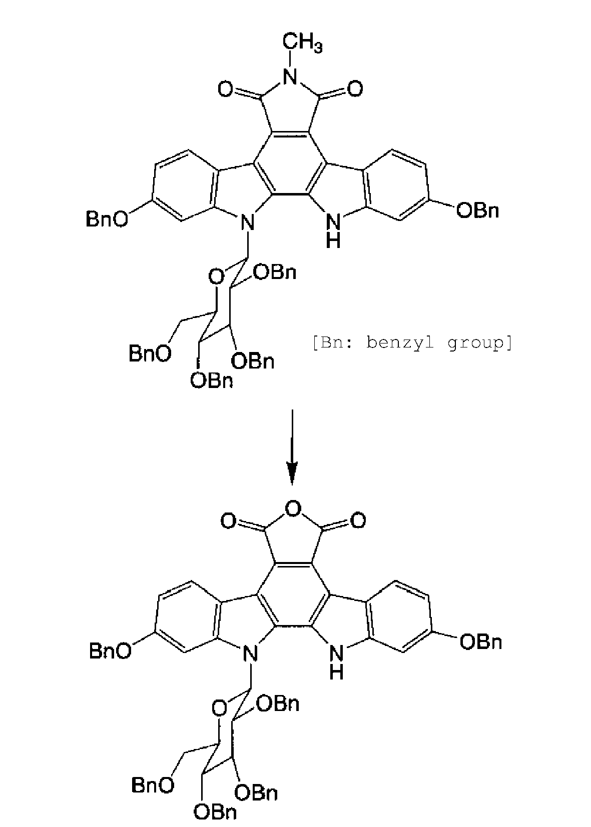

A stirrer and a thermometer were set on a 300 mL-four neck flask, and 36 mL of ethanol was placed therein. 12,13-dihydro- 2,10-dibenzyloxy-13-(β-D-2,3,4,6-tetra-O-benzylglucopyranosyl)-5H-indolo[2,3-a]pyrrolo[3,4-c]carbazole-6-methyl-5,7(6H)-dione (670 mg, 0.62 mmol) was placed therein while stirring, and the mixture was stirred at room temperature for 1 hour. A 5N-aqueous potassium hydroxide solution (8 mL) was added dropwise at the same temperature over 20 minutes. An inner temperature was set at 60°C, and then the mixture was stirred for 4 hours, and stirred at room temperature overnight. To the resulting brown solution was added toluene (20 mL), and 1.0 N hydrochloric acid (62 mL) was added dropwise at the same temperature over 30 minutes to pH 2.6. To the resulting yellow solution was added tetrahydrofuran (10 mL), and the mixture was stirred for 6 hours. The aqueous layer (lower layer) was separated, and the organic layer was washed successively with purified water (10 mL × 2) and an aqueous saturated sodium chloride solution (10 mL), dried with anhydrous sodium sulfate (5 g), and filtered. The solvent was evaporated under reduced pressure to obtain 12,13-dihydro-2,10- dibenzyloxy-13-(β-D-2,3,4,6-tetra-O-benzylglucopyranosyl)- 5H-indolo[2,3-a]carbazole-5,6-dicarboxylic acid anhydride (0.63 g; yield 85%) which is an end compound of the yellow oily residue.
Yield of end product:
Yield of end product obtained in Example 2: 93%
Yield of end product obtained in Comparative Example 2: 85%

### Content of impurities:

### Content of impurities in the end product obtained in Example 2: 0.07 area %

### Content of impurities in an end product obtained in Comparative Example 2: 0.7-2.0 area %

### High performance liquid chromatography (HPLC:measurement conditions:

Column: YMC ODS-AQ (250 × 4.6 mm)
Flow rate: 1.0 mL/min
Detection: 210 nm
Mobile phase:
0.1% aqueous H₃PO₄ solution:acetonitrile (1:99)
Injection amount: 10 µL
Temperature: 40°C

### Example 3

### Preparation of 12,13-dihydro-2,10-dibenzyloxy-6-N-(1-benzyloxymethyl-2-benzyloxyethylamino)-13-(β-D-2,3,4,6-tetra-O-benzylglucopyranosyl)-5H-indolo[2,3-a]pyrrolo[3,4-c]carbazole-5,7(6H)-dione

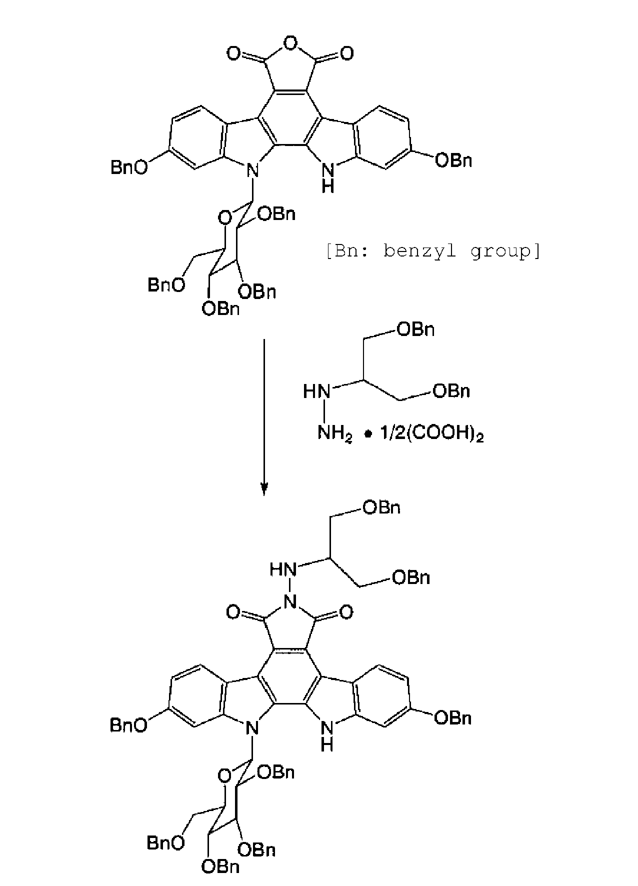

A mixture of 12,13-dihydro-2,10-dibenzyloxy-13-(β-D-2,3,4,6-tetra-O-benzylglucopyranosyl)-5H-indolo[2,3-a]carbazole-5,6-dicarboxylic acid anhydride (1.00 g, 0.94 mmol) obtained in Example 2, N-(1-benzyloxymethyl-2- benzyloxyethyl)hydrazine 1/2 oxalate (398 mg, 1.20 mmol) and N,N-dimethylacetamide (9.2 mL) was degassed, and heated to 62°C after replacement with nitrogen. To this solution was added dropwise triethylamine (0.17 mL, 1.20 mmol), and the solution was stirred at this temperature for 3 hours. The reaction solution was cooled to room temperature, and t-butyl methyl ether (20 mL) and water (4.7 mL) were added thereto. Using 1N hydrochloric acid, the pH of the aqueous layer was adjusted to 4, and the solution was stirred for 20 minutes. The organic layer was separated, washed with water (6 mL) five times, dried over sodium sulfate, and filtered. Finally, the filtrate was concentrated under reduced pressure to obtain a crude end product (1.29 g).
¹H-NMR (270 MHz, CDCl₃, δppm): 10.63(1H, br.s), 9.24(1H, br.d, J =9.6Hz), 9.16(1H, br.d, J=9.6Hz), 7.50-6.84(42H, m), 6.20(2H, br.d, J=7.6 Hz), 5.84(1H, d, J=8.6 Hz), 5.33(1H, br.d, J=3.0 Hz), 5.21(1H, d, J=12.2 Hz), 5.19(1H, d, J=11.9 Hz), 5.16(1H, d, J=12.2 Hz), 5.08 (1H, d, J=11.9 Hz), 5.08(1H, d, J=10.9Hz), 4.96(1H, d, J=10.9 Hz), 4.89(1H, d, J=10.9 Hz), 4.85(1H, d, J=10.9 Hz), 4.72(1H, d, J=12.9 Hz), 4.68(1H, d, J=12.9Hz), 4.62-4.48(4H, m), 4.33(1H, dd, J=9.6, 9.6Hz), 4.06-3.77(7H, m), 3.72(4H, d, J=5.6 Hz), 3.04(1H, d, J=9.9 Hz)
¹³C-NMR (68 MHz, CDCl₃, δppm): 168.8,168.7,159.4,159.3,143.2, 142.9,138.0,137.9,137.6,136.9,136.8,136.6,136.0,130.2,128.7,128.6,128.5,128.4,128.3,128.2,128.2,128. 1,128.0,127.9,127.8,127.7,127.6,127.5,127.4,127.3,126.9,126.6,119.4,119.1,118.0,116.9,116.7,116.1,11 0.4,96.7,96.3,85.8,84.7,80.9,77.4,77.2,76.0,75.9,75.4,74.9,73.9,73.3,73.2,70.7,70.4,69.9,69.8,66.7,58.7, 49,4,30.9,27.0

### Example 4

### Preparation of 12,13-dihydro-2,10-dihydroxy-6-N-(1-hydroxymethyl-2-hydroxyethylamino)-13-(β-D-glucopyranosyl)-5H-indolo[2,3-a]pyrrolo[3,4-c]carbazole-5,7(6H)-dione

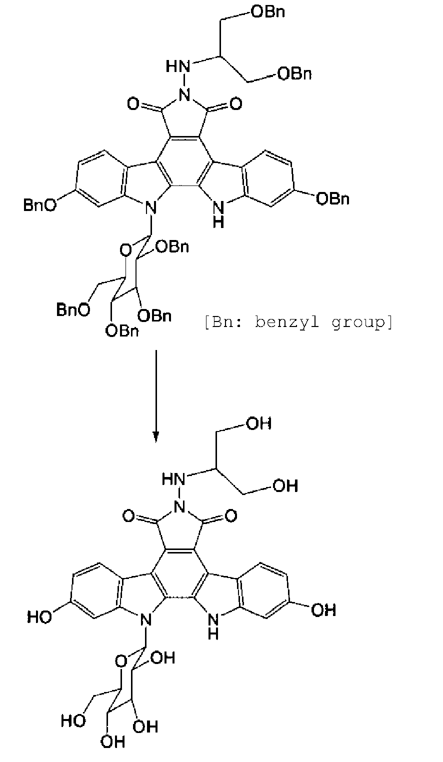

10% by weight of Pd on carbon (50% hydrous, 112 g) was filled into a 5 gallon autoclave, and a solution of 12-β-D-(2,3,4,6-tetra-O-benzylglucopyranosyl)-12,13-dihydro-2,10-dibenzyloxy-6-[[-(2-benzyloxy-1-(benzyloxymethyl)ethyl)amino]-5H-indolo[2,3-a]pyrrolo[3,4-c]carbazole-5,7(6H)-dione in tetrahydrofuran (THF) (175 g/L solution, 6.4 L, 1.12 assay kg), isopropyl alcohol (IPA, 7.9 L) and 3N HCl (224 mL) were added. The content was hydrogenated at 40°C/40 psi for 4 hours to 14 hours while rapidly stirring and, during that term, a theoretical amount of 110% by weight of hydrogen was absorbed. The content was cooled to 25°C, passed through a Solka Floc bed to filter the reaction mixture, and the filtrate was washed with 3/2 IPA/THF (1 × 3L). Using 1 M triethylamine (about 600 mL) in IPA, the filtrate was adjusted to pH 2.5, and water (4.0 L) was then added. A batch was concentrated to a 7.5 L level under atmospheric pressure. Distillation was continued at a constant batch volume while supplying 4/1 IPA/water (6.5 L). IPA (about 9 L) was supplied to a container while maintaining the batch volume at 7.5 L, to reduce a water content to 20% (w/v). The content was cooled to 70°C, and a crystal seed (5.0 g) was added as an IPA slurry (50 mL). After the batch was retained at 70°C for 1 hour, IPA (5.0 L) was added over 90 minutes. A batch was aged at 70°C for 9 to 24 hours and, during which time, the bulk of the product was crystallized. Distillation while maintaining the batch volume was performed while supplying IPA (17 L), to reduce a water content to 3% (w/v). The resulting slurry was aged at 70°C for 3 to 6 hours, cooled to 22°C, and aged for 1 hour. The slurry was filtered, and the cake was washed with IPA (2.5 L) and methanol (1.5 L) and, thereafter, dried at 38°C for 6 hours in vacuum to obtain a compound (I) as an orange solid having a purity of 99% by area or more and a yield of 80% or more.

NMR data (coupling constant (J) is described in Herz):
Regarding main rotamer δ, ¹H NMR (400.13MHz,DMSO-d₆)-data δ11.23(s, 1H), 9.80(s, 1H), 9.77(s, 1H), 8.90(d, J=8.4, 1H), 8.82(d, J=8.4, 1H), 7.21(brs, 1H), 7.01(brs, 1H), 6.84(m(overlapping), 2H), 6.00(d, J=8.0, 1H), 5.88(t, J=3.6, 1H), 5.57(d, J=2.4, 1H), 5.34(d, J =4.4, 1H), 5.13(d, J=4.4, 1H), 4.94(d, J=4.4, 1H), 4.56(t, J=5.6, 2H), 4.04(dd, J=11.2, 3.2, 1H), 3.95(m(overlapping), 2H), 3.81(dd, J=10.4, 4.0, 1H), 3.53(m(overlapping), 6H);
Regarding main rotamer δ, ¹³C NMR (100.64 MHz,DMSO-d₆)-data δ169.03,168.94,157.79,157.63,144.38,143.12,129.46,127.92,125.19(2C),118.91,117.57,115.94,114.32,1 14.23,113.92,110.30,110.24,97.54,97.49,84.49,78.39,76.77,72.88,67.53,62.59,60.47(2C),58.33.21

### Measurement conditions of High performance liquid chromatography (HPLC):

Column: YMC ODS-AQ (250 × 4.6 mm)
Flow rate: 1.5 mL/min
Detection: 228 nm
Mobile phase: A = 0.1% aqueous H₃PO₄ solution
B = acetonitrile

| Gradient: | | |
|---|---|---|
| Min. | A(%) | B(%) |
| 0 | 85 | 15 |
| 40 | 74 | 26 |
| 60 | 30 | 70 |
| 61 | 85 | 15 |
| 65 | 85 | 15 |

Injection amount: 10 µL
Temperature: 25°C

### Comparative Example 3

Using the end product obtained in Comparative Example 2, treatment was performed as in Example 3 and Example 4 to obtain an end product of Comparative Example 3.

### Impurities content:

### Content of impurities in the end product obtained in Example 4: 0.2% by area

### Content of impurities in the end product obtained in Comparative Example 3: 0.5 to 1.0% by area

### Measurement conditions of High performance liquid chromatography (HPLC):

Column: YMC ODS-AQ (250 × 4.6 mm)
Flow rate: 1.5 mL/min
Detection: 228 nm
Mobile phase: A = 0.1 % aqueous H3PO4 solution
B = acetonitrile

| Gradient: | | |
|---|---|---|
| Min. | A(%) | B(%) |
| 0 | 82 | 18 |
| 10 | 78 | 22 |
| 15 | 78 | 22 |
| 28 | 30 | 70 |
| 29 | 82 | 18 |
| 35 | 82 | 18 |

Injection amount: 10 µL
Temperature: 50°C

### INDUSTRIAL APPLICABILITY

According to the process of the present invention, a compound useful as an anti-cancer agent in the pharmaceutical field and an intermediate for producing the same can be produced at high quality and efficiently.

## Claims

1. A process for producing an indolopyrrolocarbazole derivative represented by the formula (I): 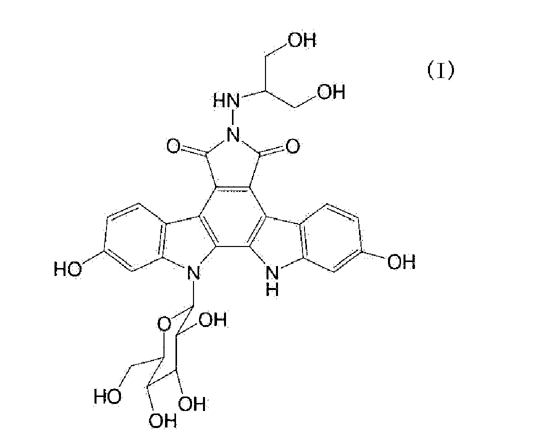 or a pharmaceutically acceptable salt thereof, which comprises:
(i) the step of treating a compound represented by the formula (V): 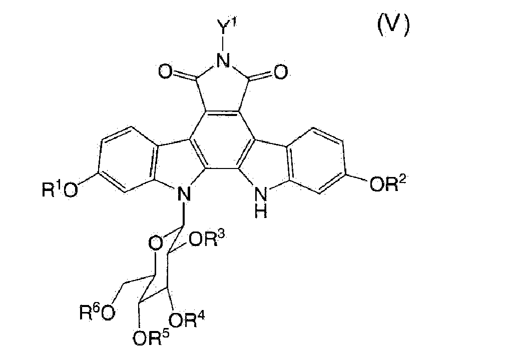 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group, or a solvate thereof or a salt thereof, with a base in an inert solvent, followed by treatment with an acid;
(ii) the step of further treating the resulting reaction solution with a base in an inert solvent and subsequently with an acid to obtain a compound represented by the formula (IV): 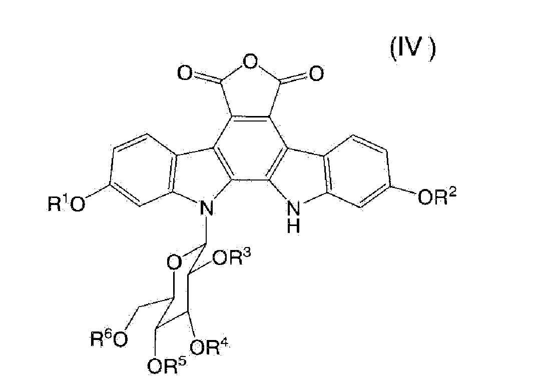 wherein R¹, R², R³, R⁴, R⁵ and R⁶ each independently represent a hydroxy protecting group, or salt thereof;
(iii) the step of reacting the resulting compound of the formula (IV) with hydrazine diol represented by the formula (III): 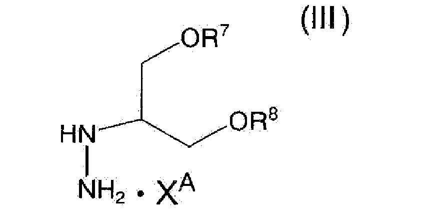 wherein X^{A} represents no existence of any molecule or an acid molecule; and R⁷ and R⁸ each independently represent a hydrogen atom or a hydroxy protecting group, or an acid addition salt thereof in the presence of an acid scavenger to obtain a compound represented by the formula (II): 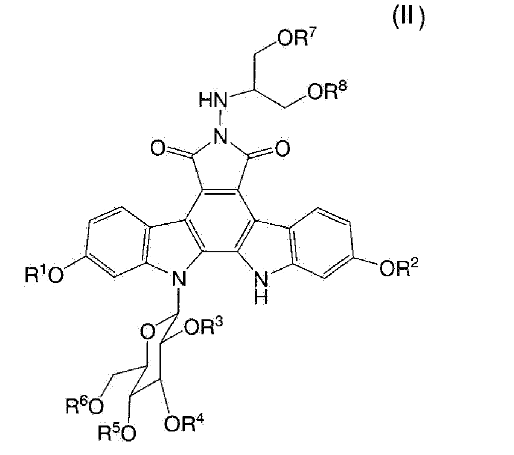 wherein R¹, R², R³, R⁴, R⁵ and R⁶ each independently represent a hydroxy protecting group; and R⁷ and R⁸ each independently represent a hydrogen atom or a hydroxy protecting group, or a salt thereof; and
(iv) the step of removing the protecting groups from the resulting compound of the formula (II).

2. The process according to claim 1, wherein a solvate or a salt of the compound represented by the formula (V) is a compound represented by the formula (VI): 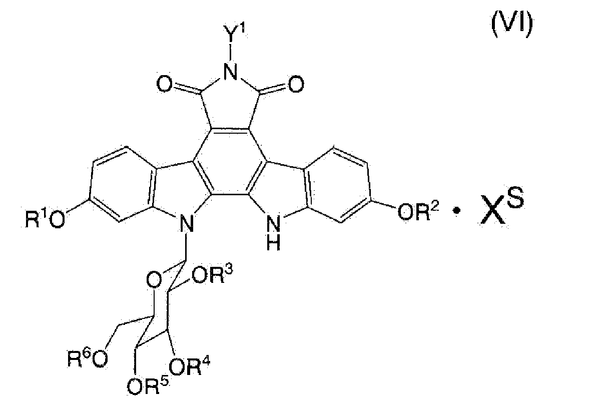 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently have the same meaning as defined below, or a salt thereof, which is obtained by carrying out the following steps (i) and (ii):
(i) the step of reacting a compound represented by the formula (VIII): 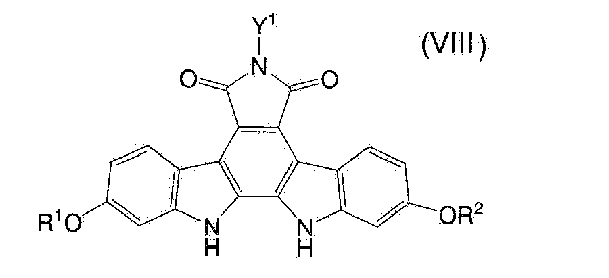 wherein Y¹ is defined above, R¹ and R² each independently represent a hydroxy protecting group, or a salt thereof, with a compound represented by the formula (VII): 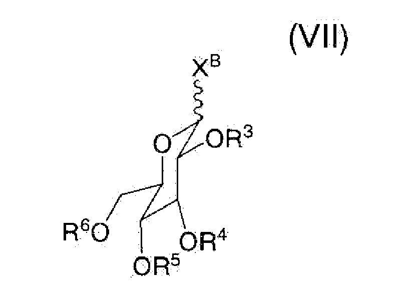 wherein X^{B} represents a halogen atom, and R³, R⁴, R⁵ and R⁶ each independently have the same meaning as defined above, in the presence of a base and a phase transfer catalyst; and
(ii) the step of crystallizing the reaction product obtained in the above step as a solvate or a salt thereof.

3. The process according to claim 1, wherein a solvate of the compound represented by the formula (V) is a compound represented by the formula (VI): 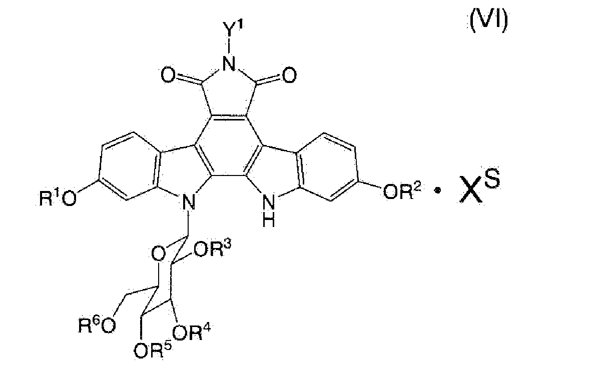 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.

4. The process according to any one of claims 1 to 3, wherein R¹, R², R³, R⁴, R⁵, R⁶, R⁷ and R⁸ each represent a benzyl group.

5. The process according to any one of claims 1 to 4, wherein X^{A} is 1/2 oxalic acid.

6. The process according to any one of claims 1 to 5, wherein Y¹ is a methyl group.

7. A solvate compound represented by the formula (VI): 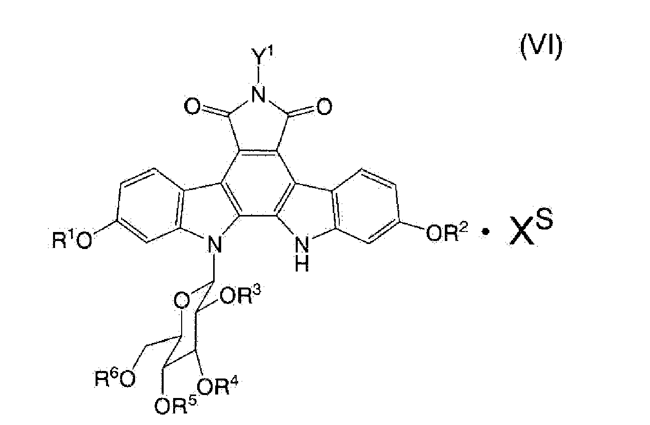 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵, and R⁶ each independently represent a hydroxy protecting group, or a salt thereof.

8. The compound or a salt thereof according to claim 7, wherein the solvate compound represented by the formula (VI) is a compound represented by the formula (VIa): 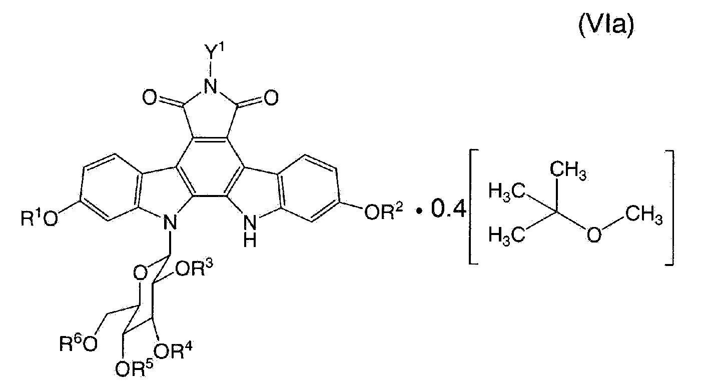 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.

9. The compound or a salt thereof according to claim 7 or 8, wherein Y¹ is a methyl group, and R¹, R², R³, R⁴, R⁵ and R⁶ each represent a benzyl group.

10. A process for producing a solvate compound represented by the formula (VI): 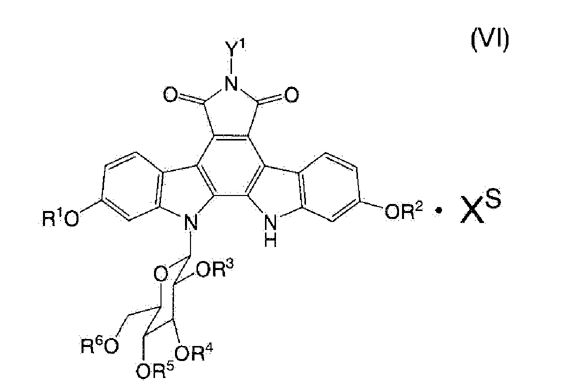 wherein X^{S} represents a solvent molecule; Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group, or a salt thereof, which comprises:
(i) the step of reacting a compound represented by the formula (VIII): 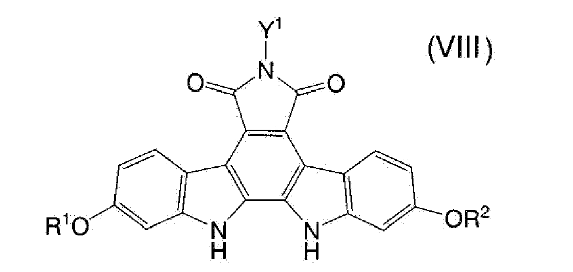 wherein Y¹ represents a hydrogen atom, a C₁-C₄ alkyl group, a phenyl group, a benzyloxymethyl group or an aralkyl group; and R¹ and R² each independently represent a hydroxy protecting group, or a salt thereof, with a compound represented by the formula (VII): 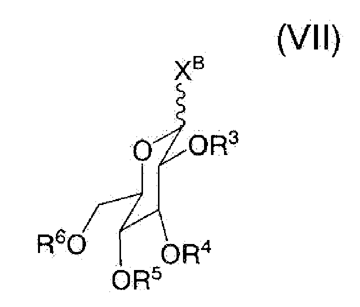 wherein X^{B} represents a halogen atom; and R³, R⁴, R⁵ and R⁶ each independently represent a hydroxy protecting group in the presence of a base and a phase transfer catalyst; and
(ii) the step of crystallizing the reaction product obtained in the above step into a solvate or a salt thereof.

11. The process according to claim 10, wherein the compound represented by the formula (VI) which is a solvate is a compound represented by the formula (VIa): 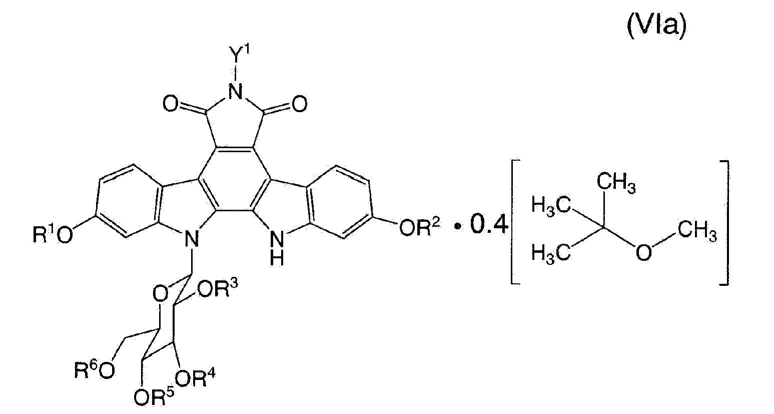 wherein Y¹ represents a hydrogen atom, a C₁-C₄ alkyl group, a phenyl group, a benzyloxymethyl group or an aralkyl group; and R¹, R², R³, R⁴, R⁵ and R⁶ each independently represent a hydroxy protecting group.

12. The process according to claim 10 or 11, wherein Y¹ is a methyl group, and R¹, R², R³, R⁴, R⁵ and R⁶ each represent a benzyl group.

13. A process for producing a compound represented by the formula (IV): 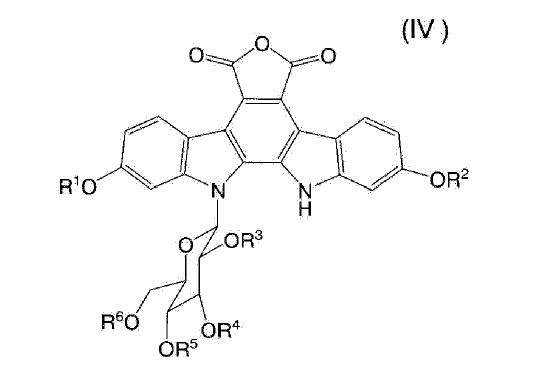 wherein R¹, R², R³, R⁴, R⁵ and R⁶ each independently represent a hydroxy protecting group, or a salt thereof, which comprises:
(i) the step of treating a compound represented by the formula (V): 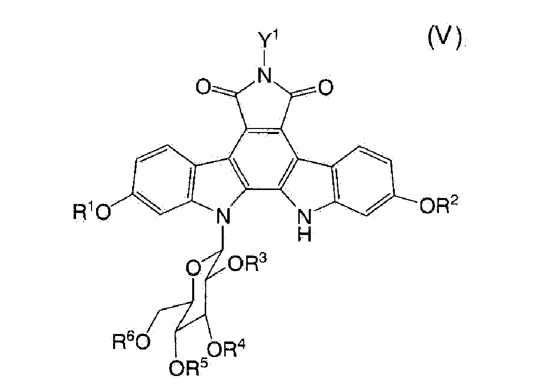 wherein Y¹ represents a hydrogen atom, a C₁-C₄ alkyl group, a phenyl group, a benzyloxymethyl group or an aralkyl group; and R¹, R², R³, R⁴, R⁵, and R⁶ each independently represent a hydroxy protecting group, or a salt thereof, with a base in an inert solvent, followed by treatment with an acid; and
(ii) the step of treating the reaction solution obtained in the above step with a base in an inert solvent and subsequently with an acid.

14. The process according to claim 13, wherein R¹, R², R³, R⁴, R⁵ and R⁶ each represent a benzyl group.
